# EUROPÄISCHE PATENTANMELDUNG

(11) **EP 3 754 333 A1**
(43) Veröffentlichungstag der Anmeldung: **23.12.2020**
(21) Anmeldenummer: 19181241.1
(22) Anmeldetag: 19.06.2019
(51) Int. Cl.: G01N 33/36, D01H 13/32, G01N 21/88

(54) **VORRICHTUNG ZUR MESSUNG VON KENNWERTEN EINES FADENPRÜFGUTES SOWIE SYSTEM UMFASSEND EINE VORRICHTUNG ZUR MESSUNG VON KENNWERTEN EINES FADENPRÜFGUTES UND EIN FADENPRÜFGERÄT**

(71) Anmelder: TEXTECHNO HERBERT STEIN GMBH & CO. KG, 41066 Mönchengladbach (DE)
(72) Erfinder: Fliescher, Stefan Dr., 41066 Mönchengladbach (DE); Mörschel, Ulrich Dr., 41066 Mönchengladbach (DE); Bönnen, Thomas, 41066 Mönchengladbach (DE)
(74) Vertreter: dompatent von Kreisler Selting Werner - Partnerschaft von Patent- und Rechtsanwälten mbB

(57) **Zusammenfassung**

Die Erfindung betrifft eine Vorrichtung zur Messung von Kennwerten eines Fadenprüfgutes mit einem Messkanal mit zwei gegenüberliegenden Seitenwänden und einer Rückwand zur kontinuierlichen Durchführung des Fadenprüfgutes entlang dessen Fadenlängsachse, einer Beleuchtungseinrichtung, einer elektronischen Bildsensoreinrichtung sowie einer Auswerteeinrichtung für die Signale der Bildsensoreinrichtung.

## Beschreibung

Die vorliegende Erfindung betrifft eine Vorrichtung zur Messung von Kennwerten eines Fadenprüfgutes gemäß den Merkmalen des Oberbegriffs des Anspruchs 1 und betrifft weiterhin ein System umfassend eine Vorrichtung zur Messung von Kennwerten eines Fadenprüfgutes und ein Fadenprüfgerät gemäß den Merkmalen des Anspruchs 15.

Aus dem Stand der Technik sind bereits Vorrichtungen zur Messung von Kennwerten eines Fadenprüfgutes bekannt geworden, welche einen Messkanal mit zwei gegenüberliegenden Seitenwänden und eine Rückwand zur kontinuierlichen Durchführung des Fadenprüfgutes entlang dessen Faserlängsachse, eine Beleuchtungseinrichtung, eine elektronische Bildsensoreinrichtung sowie eine Auswerteeinrichtung für die Signale der Bildsensoreinrichtung umfasst.

Nachteilig an den Vorrichtungen zur Messung von Kennwerten eines Fadenprüfgutes gemäß dem vorgenannten Stand der Technik ist, dass die bekannten Vorrichtungen über aufwändige Optiken im Bereich der Bildsensoreinrichtung sowie ggf. der Beleuchtungseinrichtungen verfügen. Die optischen Einrichtungen wie beispielsweise Linsen mit einer hohen optischen Qualität zur Gewährleistung einer verzerrungsfreien Abbildung zunächst den Nachteil aufweisen, dass diese sehr kostenintensiv sind, in der Regel eine Justage erfordern und folglich die Gesamtkosten der Messvorrichtung negativ beeinflussen.

Weiterhin ist es notwendig, die vorbekannten Vorrichtungen zur Messung von Kennwerten eines Fadenprüfgutes aufgrund der eingesetzten Optiken, insbesondere im Bereich der Bildsensoreinrichtungen, aufwändig zu kalibrieren, um valide und vergleichbare Messwerte für die Kennwerte des Fadenprüfgutes zu erhalten. Weiterhin weisen die vorbekannten Vorrichtungen den zusätzlichen Nachteil auf, dass die verwendeten Optiken meist störungsempfindlich gegenüber Verschmutzungen des überprüften Fadenprüfgutes sowie gegenüber von Veränderungen der Umgebungsbedingungen, wie beispielsweise der Messtemperatur und der Luftfeuchtigkeit, sein können.

Ausgehend von dem vorbekannten Stand der Technik besteht die Aufgabe der vorliegenden Erfindung darin, eine Vorrichtung zur Messung von Kennwerten eines Fadenprüfgutes bereitzustellen, die keine zusätzliche Kalibrierung erfordert und die weiterhin gegenüber äußeren Einflüssen wie beispielsweise Temperaturänderungen und Verunreinigungen unempfindlich ist und damit eine höhere Messgenauigkeit ermöglicht.

Erfindungsgemäß gelöst wird die vorgenannte Aufgabe durch eine Vorrichtung zur Messung von Kennwerten eines Fadenprüfgutes, welche dadurch gekennzeichnet ist, dass die Beleuchtungseinrichtung und die Bildsensoreinrichtung in einem ersten Abstand einander gegenüberliegend angeordnet sind, wobei die Beleuchtungseinrichtung und die Bildsensoreinrichtung zumindest abschnittsweise die gegenüberliegenden Seitenwände eines Messkanalabschnittes bilden und dass die Beleuchtungseinrichtung unmittelbar ein Schattenbild des Fadenprüfgutes auf die Bildsensoreinrichtung wirft, wobei mindestens ein Schattenbild des Fadenprüfgutes an die Auswerteeinrichtung zum Bestimmen mindestens eines Haarigkeitskennwertes und/oder Durchmesserwertes des Fadenprüfgutes übertragen wird.

Die erfindungsgemäße Ausführungsform weist dabei den Vorteil auf, dass durch den Verzicht auf Optiken in dem Bereich zwischen Beleuchtungseinrichtung und der Bildsensoreinrichtung eine Kalibrierung der erfindungsgemäßen Vorrichtung nicht erforderlich ist. Weiterhin ist die erfindungsgemäß ausgestaltete Messvorrichtung unempfindlich gegenüber äußeren Störgrößen, da auf zusätzliche Optiken im Strahlengang zwischen Beleuchtungseinrichtung und Bildsensoreinrichtung verzichtet wird. Weiterhin wird das Schattenbild zur Generierung der Bilddaten unmittelbar auf die elektronische Bildsensoreinrichtung projiziert bzw. unmittelbar in der Sensorebene der elektronischen Bildsensoreinrichtung erzeugt, erfindungsgemäß kann somit auf die Anordnung von zusätzlichen Ebenen oder optischen Einrichtungen zur Abbildung des zu charakterisierenden Fadenprüfgutes verzichtet werden, womit weitere Fehlerquellen für die Messung entfallen.

Bei der Bildsensoreinrichtung handelt es sich um eine optikfreie Bildsensoreinrichtung, wobei unter dem Begriff "optikfrei" im Rahmen der vorliegenden Anmeldung verstanden wird, dass mit Ausnahme einer geringfügigen Parallelverschiebung beim Durchgang durch ggf. planparallele Fenster, welche der Bildsensoreinrichtung im Strahlengang vorgelagert werden, keine optische Beeinflussung der Lichtstrahlen bzw. des Strahlengangs zwischen der Beleuchtungseinrichtung und der Bildsensoreinrichtung vorgenommen wird. Insbesondere findet keine Beeinflussung des Strahlengangs durch Linsen statt.

Unter dem Begriff des Fadenprüfgutes wird im Rahmen der vorliegenden Erfindung beispielsweise ein Einzel- bzw. Monofilament oder ein Multifilament bestehend aus einer Vielzahl von Einzelfilamenten verstanden, insbesondere kann es sich um ein aus Stapelfasern gesponnenes Garn in seinen verschiedenen Varianten, wie beispielsweise Einfachgarn, gefachtes Garn, Zwirn oder Core-Garn, handeln. Bei den Monofilamenten ist es vorgesehen, dass erfindungsgemäß als Kennwert lediglich die Dicke bzw. der Durchmesser des Fadenkerns ermittelt wird.

Das Fadenprüfgut wird mit einer Prüfgeschwindigkeit durch den Messkanal bewegt. Die Geschwindigkeit kann dabei verändert werden, bevorzugt liegen die Prüfgeschwindigkeiten der erfindungsgemäßen Vorrichtung im Bereich bis 2000 m/min, besonders bevorzugt liegt die Geschwindigkeit im Bereich von 25 bis 800 m/min, insbesondere im Bereich von 400 bis 800 m/min. Die mit der Vorrichtung erreichbaren hohen Prüfgeschwindigkeiten ermöglichen eine Charakterisierung des Fadenprüfgutes gleichzeitig mit anderen Messverfahren, bei denen mit den vorgenannten Geschwindigkeiten geprüft wird, wie zum Beispiel der kapazitiven Messung der Massenungleichmäßigkeit des Fadenprüfgutes bzw. Garnes.

Das Fadenprüfgut weist einen Fadenkern auf, von dem eine Vielzahl von Einzelfasern (Haaren) abstehen.

Im Rahmen der vorliegenden Erfindung werden folgende Eigenschaften als Haarigkeitswerte verstanden, welche unter Zuhilfenahme der erfindungsgemäßen Vorrichtung bestimmt werden können:
- Länge der von dem Fadenkern abstehenden einzelnen Fasern (Haare),
- die Gesamthaarigkeit, definiert als die Summe der Längen der von dem Fadenkern abstehenden Fasern pro Längeneinheit des Fadenprüfgutes, wobei diesbezüglich weiterhin die statistischen Kennwerte der Gesamthaarigkeit wie insbesondere der Mittelwert, die Variationskoeffizienten, die Standardabweichung, das Minimum, das Maximum sowie ein Spektrogramm ermittelt werden können,
- die Haarigkeit definiert als die Summe der Längen der von dem Fadenkern abstehenden Fasern pro Längeneinheit des Fadenprüfgutes als Funktion des Abstandes vom Fadenkern,
- die Häufigkeit bzw. Anzahl des Auftretens von Haarigkeitsfasern in bestimmten Abständen zu dem Fadenkern pro Bezugslänge,
- weiterhin kann jedoch auch der Durchmesser des Fadenkerns des Fadenprüfgutes in einer zweidimensionalen Projektion als Dicke gemessen werden, dabei können weiterhin die statistischen Kennwerte des Durchmessers des Fadenkerns, wie insbesondere der Mittelwert, die Variationskoeffizienten sowie die Standardabweichung, das Minimum und Maximum sowie das Spektrogramm ermittelt werden.

Der Messkanal kann erfindungsgemäß eine Kanalrückwand umfassen, deren Oberfläche im Wesentlichen orthogonal zu den beiden Seitenwänden und parallel zu der Fadenlängsachse angeordnet ist, so dass die Kanalrückwand den Messkanal zur Rückseite hin abschließt bzw. begrenzt. Die Kanalrückwand bildet dabei in Kombination mit den gegenüberliegenden Seitenflächen einen nach vorne offenen Messkanal. Bei der optionalen Ausgestaltung des Messkanals mit planen Wänden entsteht ein rechteckiger Messkanalquerschnitt, welcher gegenüberliegend der Kanalrückwand offen ausgestaltet ist, die Seitenwände und die Rückwand formen im Querschnitt des Messkanals eine U-förmige Messkanalwandung.

Über die Öffnung des Messkanals entlang der gesamten Vorderseite des Messkanals kann erreicht werden, dass das Fadenprüfgut nicht mit seinem Anfang in den Messkanal und die Messvorrichtung eingefädelt werden muss. Es ergibt sich daher der Vorteil, dass beispielsweise die Messvorrichtung bei stehendem oder ebenfalls bei bewegtem Fadenprüfgut über dieses geschoben oder geschwenkt werden kann.

Alternativ kann das Fadenprüfgut beispielsweise über verlagerbare Fadenleitelemente bzw. ein verlagerbares Fadenförderwerk in den Messkanal der Messvorrichtung verlagert werden. Eine Einbringung des Fadenprüfgutes in den Messkanal der Messvorrichtung ist damit problemlos ebenfalls bei bewegtem Fadenprüfgut möglich. Der Messkanal weist damit einen Schlitz gegenüberliegend der Messkanalrückwand auf.

Insbesondere kann bei einem vorliegenden endlosen Fadenprüfgut mit einem bereits bestehenden freien Fadenlauf, beispielsweise in einer Textilverarbeitungsmaschine oder in einem Fadenprüfgerät, das Fadenprüfgut in den Messkanal der erfindungsgemäßen Messvorrichtung durch Verschieben und/oder Verschwenken der Messvorrichtung relativ zu dem freien Fadenlauf in den Messkanal eingebracht werden und es kann eine berührungslose Messung der Haarigkeitskennwerte bzw. der Dicke des Fadenkerns mittels der Messvorrichtung erfolgen. Die vorgenannte Ausführungsform weist dabei den Vorteil auf, dass der bestehende freie Fadenlauf nicht unterbrochen oder das fadenförmige Prüfgut zur Vornahme der Messung nicht angehalten werden muss. Insbesondere findet durch die erfindungsgemäße Messvorrichtung keine Beeinflussung des freien Fadenlaufes während der Messung statt. Folglich kann die erfindungsgemäße Vorrichtung in allen bereits bestehenden Fadenverarbeitungsmaschinen oder Fadenprüfgeräten eingesetzt werden, da durch die erfindungsgemäße Vorrichtung keine Beeinflussung der übrigen Komponenten erfolgt.

Erfindungsgemäß kann der Ablauf der Kennwertbestimmung in der Auswerteeinrichtung wie folgt vorgenommen werden:
- Identifikation des Kerns des Fadenprüfguts (durch zweidimensionale Glättung zur Unterdrückung der abstehenden Einzelfasern) und anschließende Schwellwertbildung,
   - Identifikation der abstehenden Einzelfasern durch Kantendetektion,
   - Ausblenden des Fadenkerns,
   - Berechnung des Abstandes jedes Punktes der abstehenden Einzelfasern zum Fadenkern,
   - Verfolgung jedes Punktes einer abstehenden Einzelfaser vom Fadenkern bis zum Einzelfaserende.

Bei einem bevorzugten Ausführungsbeispiel ist es vorgesehen, dass in Bewegungsrichtung des Fadenprüfgutes vor dem Messkanal eine Bremseinrichtung für das Fadenprüfgut und/oder hinter dem Messkanal eine Fördereinrichtung für das Fadenprüfgut angeordnet sind.

Durch die Anordnung einer Bremseinrichtung vor dem Messkanal und/oder einer Fördereinrichtung für das Fadenprüfgut hinter dem Messkanal kann insbesondere der Spannungszustand des Fadenprüfguts im Bereich des Messkanals der Vorrichtung zur Messung der Kennwerte des Fadenprüfgutes beliebig eingestellt bzw. beeinflusst werden.

Alternativ kann jedoch auch eine Ausgestaltung vorgesehen werden, bei welcher in Bewegungsrichtung des Fadenprüfgutes hinter dem Messkanal eine Absaugeinrichtung für das Fadenprüfgut und vor dem Messkanal eine Fördereinrichtung für das Fadenprüfgut angeordnet sind.

Als Bremseinrichtung können beispielsweise eine Fadenbremse oder ein Fournisseur, als Fördereinrichtung ein Fadenförderer wie beispielsweise ein Walzenlieferwerk, Casablanca-Riemchenlieferwerk, oder ein Galettenlieferwerk und als Absaugeinrichtung eine druckluftbetriebene Düse, insbesondere Coanda-Düse, vorgesehen werden.

Die Bremseinrichtung, die Fadenfördereinrichtung und/oder eine Fadenleiteinrichtung, welche betrachtet in Fadenlängsrichtung vor und/oder hinter dem Messkanal der Messvorrichtung angeordnet werden können, können derart verlagerbar, insbesondere verschiebbar und/oder verschwenkbar, ausgestaltet werden, dass eine Verlagerung des Fadenprüfguts im Wesentlichen orthogonal zu dessen Fadenlängsachse relativ zu dem Messkanal realisiert werden kann. Die vorgenannten verlagerbaren Einrichtungen für das Fadenprüfgut können dementsprechend genutzt werden, um bei ortsfester Ausgestaltung der erfindungsgemäßen Vorrichtung zur Messung von Kennwerten des Fadenprüfguts das Fadenprüfgut von einer Ruheposition, in welcher das Fadenprüfgut beabstandet zu der erfindungsgemäßen Vorrichtung angeordnet ist bzw. verläuft, in eine Messposition verlagert werden, in welcher das Fadenprüfgut im Messkanal verläuft.

Ebenfalls kann eine Verlagerung des Fadenprüfgutes in dem Messkanal zur Realisierung einer beliebigen Positionierung des Fadenprüfguts im Querschnitt des Messkanals und insbesondere der Messkanalwände vorgenommen werden. So kann es insbesondere vorgesehen werden, dass die Lage des Fadenprüfguts innerhalb des Messkanals variiert werden kann, vorzugsweise parallel zu den Wänden des Messkanals. Diese Ausgestaltung weist den Vorteil auf, dass durch das laufende bzw. bewegte Fadenprüfgut der Messkanal bzw. die Messkanalwände gereinigt werden können, indem beispielsweise der Messkanal periodisch mit dem Fadenprüfgut durchstrichen bzw. die Messkanalwände überstrichen werden.

In einer bevorzugten Ausführungsform ist vorgesehen, dass die Beleuchtungseinrichtung und die gegenüberliegende elektronische Bildsensoreinrichtung jeweils eine dem Messkanal zugewandte plane Oberfläche aufweisen, wobei die gegenüberliegenden Oberflächen im Wesentlichen parallel zu der Fadenlängsachse angeordnet sind, derart, dass zumindest zwischen den Oberflächen der Bildsensoreinrichtung und der Beleuchtungseinrichtung ein Messkanalabschnitt mit einer gleichbleibenden ersten Kanalbreite ausgebildet wird.

Die erste gleichbleibende Kanalbreite entspricht dabei dem ersten Abstand zwischen den beiden gegenüberliegenden Seitenwänden des Messkanalabschnitts. Die planen Oberflächen der Bildsensoreinrichtung und/oder der Beleuchtungseinrichtung können jeweils durch eine transparente Scheibe, insbesondere eine Saphirglasscheibe gebildet werden, dabei kann es vorgesehen sein, dass die Scheibe entspiegelt ausgestaltet wird, wobei die Entspiegelung insbesondere auf der dem Messkanal zugewandten und/oder abgewandten Oberfläche vorgesehen werden kann. Die Scheiben dienen dabei nicht der Beeinflussung des Strahlengangs, vielmehr dienen diese lediglich dazu die Elemente der Beleuchtungseinrichtung und der elektronischen Bildsensoreinrichtung von dem Fadenprüfgut abzuschirmen. Durch die hohen Prüfgeschwindigkeiten in Verbindung mit den Eigenschaften des Fadenprüfgutes verhindern die Scheiben ein Verschmutzen oder Verkratzen sowohl der Belichtungs- als auch der elektronischen Bildsensoreinrichtung.

Nach einer besonders bevorzugten Ausführungsform ist es vorgesehen, dass in Bewegungsrichtung des Fadenprüfgutes vor dem Messkanalabschnitt zusätzlich ein vorderer Kanalabschnitt ausgestaltet ist, wobei sich der Abstand zwischen den gegenüberliegenden Seitenwänden in dem vorderen Kanalabschnitt von einem initialen Abstand an einer Eintrittsseite hin zu dem ersten Abstand im Bereich der an den Messkanalabschnitt angrenzenden Austrittsseite des vorderen Kanalabschnitts verjüngt. Der initiale Abstand ist dabei größer als der erste Abstand. Die Eintrittsseite des vorderen Kanalabschnittes ist betrachtet in Fadenlängsrichtung dem Messkanalabschnitt abgewandt.

Der vordere Kanalabschnitt kann ebenfalls eine Rückwand aufweisen, wobei die Rückwand des vorderen Kanalabschnitts bevorzugt mit der optionalen Rückwand des Messkanalabschnitts eine Ebene bzw. Fläche bildet.

In einer alternativen Ausführungsform kann die Rückwand im Bereich des vorderen Kanalabschnittes jedoch ebenfalls einen konvexen Oberflächenverlauf aufweisen, derart, dass sich der Abstand zwischen der Oberfläche der Rückwand und der Fadenlängsachse von dem halben Wert des initialen Abstands im Bereich des Eintrittsendes des vorderen Messkanalabschnitts hin zu dem halben Wert des ersten Abstandes an der Austrittsseite des vorderen Messkanalabschnitts verkleinert.

Insbesondere kann es vorgesehen sein, dass im Bereich der Eintrittsseite des vorderen Kanalabschnitts an mindestens einer der beiden gegenüberliegenden Seitenwände eine Ausströmöffnung ausgebildet ist, über welche ein Gasvolumenstrom parallel zu der Oberfläche der jeweiligen Seitenwand im Bereich der Eintrittsseite, bevorzugt orthogonal zu der Längsachse des Fadenprüfguts ausstoßbar ist.

In einer bevorzugten Ausführungsform ist es dabei vorgesehen, dass der Verlauf von mindestens einer der Wände im Bereich des vorderen Kanalabschnitts derart ausgestaltet ist, dass diese eine Wechselwirkung mit dem über die Ausströmöffnung ausgestoßenen Gasvolumenstrom ausübt, derart, dass der Gasvolumenstrom entlang der mindestens einen entsprechend ausgestalteten Wand des vorderen Kanalabschnitts von einer zunächst schräg, bevorzugt orthogonal zu der Fadenlängsachse verlaufenden, Bewegungsrichtung im Bereich der Ausströmöffnung in eine im Wesentlichen parallel zu der Fadenlängsachse verlaufende Bewegungsrichtung im Bereich des Austrittsendes des vorderen Kanalabschnitts umgelenkt wird. Als Wechselwirkung kann dabei der sogenannte Coanda-Effekt genutzt werden, im Rahmen dessen der durch die Ausströmöffnung ausgestoßene Gasvolumenstrom als räumlich eng begrenzte Strömung, die sich von der ruhenden Umgebung deutlich unterscheidet, an der gekrümmten Oberfläche der Seitenwand des Messkanals entlangläuft und dabei dem Oberflächenverlauf der Seitenwand folgt. Es bildet sich folglich ein Gasvolumenstrom, welcher parallel zu der Fadenlängsachse verläuft und das Fadenprüfgut mindestens einseitig überstreicht oder aber allseitig umhüllt. Die sich aufgrund der Fadenprüfgeschwindigkeit anlegenden Fasern bzw. Haare des Fadenprüfgutes können durch den in Bewegungsrichtung des Fadenprüfgutes verlaufenden und das Fadenprüfgut umhüllenden Gasvolumenstrom wieder aufgerichtet werden.

Bei einer bevorzugten Ausführungsform kann es vorgesehen sein, dass mindestens eine der Wände im Bereich der Eintrittsseite des vorderen Kanalabschnitts schräg, bevorzugt orthogonal, zu der Fadenlängsachse und im Bereich der Austrittsseite des vorderen Kanalabschnitts im Wesentlichen parallel zu der Fadenlängsachse verläuft.

In einer weiterhin bevorzugten Ausführungsform ist es vorgesehen, dass mindestens eine der Wände im Bereich des vorderen Kanalabschnitts gekrümmt ausgestaltet ist, wobei die mindestens eine gekrümmte Wand einen konvexen Oberflächenverlauf aufweist.

Der Verlauf der Seitenwand kann im vorderen Kanalabschnitt bevorzugt einen Kreisabschnitt, bevorzugt einen Viertelkreis mit konstantem Radius, aufweisen.

Es kann vorgesehen sein, dass pro definierter Zeitdauer das ausgestoßene Volumen des über die mindestens eine Ausströmöffnung ausgestoßenen Gases veränderbar ist. Durch die Veränderung des pro Zeitdauer ausgestoßenen Volumens ist es möglich, die Strömungsverhältnisse innerhalb des Messkanals anzupassen. Durch die gezielte Beeinflussung des ausgestoßenen Volumens je Zeitdauer kann sichergestellt werden, dass stets eine laminare Strömung im Messkanal vorliegt.

Der ausgestoßene Volumenstrom kann dabei bevorzugt an die Geschwindigkeit des Fadenprüfguts angepasst werden, so dass bevorzugt der das Fadenprüfgut umhüllende Fluidstrom beispielsweise die gleiche Geschwindigkeit wie das Fadenprüfgut aufweist. Diese Ausgestaltung hat den Vorteil, dass sich die aufgrund des auftretenden Luftwiderstands an den Fadenkern anlegenden Fasern (Haare) des Fadenprüfguts wieder aufrichten und somit trotz hoher Prüfgeschwindigkeit das gleiche Messergebnis für die Kennwerte der Haarigkeit wie bei langsamen Geschwindigkeiten bzw. wie im Ruhezustand des Fadenprüfguts gemessen werden können. Weiterhin weist das ausgeströmte Volumen den Vorteil auf, dass der Messkanal durch das ausgeströmte Volumen gespült wird, so dass sich keine durch das Fadenprüfgut mitgeführten Feststoffpartikel in dem Messkanal absetzen bzw. dort vorliegende Partikel mit dem Volumenstrom aus dem Bereich des Messkanals ausgespült werden können, wo mittels des ausgeströmten Volumenstroms der Messkanal dauerhaft sauber gehalten werden kann, ohne manuelle Eingriffe in der Optik der erfindungsgemäßen Messvorrichtung vornehmen zu müssen.

In einer weiter besonders bevorzugten Ausführungsform ist es vorgesehen, dass die Beleuchtungseinrichtung durch eine transparente Scheibe und eine in einem definierten Abstand zu der transparenten Scheibe angeordneten Lichtquelle ausgebildet ist. Bei der transparenten Scheibe kann es sich beispielsweise um eine Glasscheibe handeln.

Der Abstand der Lichtquelle zu dem Prüfgut muss groß in Relation zu dem Abstand des Fadenprüfguts zu der Bildsensoreinrichtung sein.

Bei der Lichtquelle kann es sich beispielsweise um eine lichtemittierende Diode handeln, die Lichtquelle kann dabei elektromagnetische Strahlung im gesamten Wellenlängenbereich von infraroter bis ultravioletter Strahlung (1 nm bis 10⁶ nm Wellenlänge aufweisen), bevorzugt im sichtbaren Wellenlängenbereich (380 nm bis 750 nm), aufweisen.

In einer weiterhin bevorzugten Ausführungsform wird eine Beleuchtungseinrichtung verwendet, welche inkohärentes Licht aussendet. Dieses weist den Vorteil auf, dass im Schattenbild des Fadenprüfgutes, welches auf der Bildsensoreinrichtung abgebildet wird keine Beugungsmuster erzeugt werden, welche die Ermittlung der Haarigkeitskennwerte und des Fadendurchmessers erschweren würden.

Besonders bevorzugt kann blaues Licht beispielsweise mit einer Wellenlänge von 455 nm von der Leuchtquelle ausgesendet werden.

Die Lichtquelle kann dabei eine Strahlungsleistung im Bereich von 50 mW bis 5000 mW, bevorzugt im Bereich von 500 mW bis 2000 mW, aufweisen.

Gemäß einer weiterhin bevorzugten Ausführungsform kann es vorgesehen sein, dass die Bildsensoreinrichtung eine Mehrzahl an Bildern von dem Fadenprüfgut mit einer definierten Bildwiederholrate aufnimmt und zur Bestimmung von Haarigkeitskennwerten an die Auswerteeinrichtung überträgt. Durch die Aufnahme von einer Vielzahl an Bildern von dem Fadenprüfgut können für dieses statistisch verlässliche Messwerte der Haarigkeitskennwerte und/oder des Fadendurchmessers ermittelt werden. Weiterhin können jedoch auch auftretende Änderungen der Fadenkennwerte entlang der Fadenlängsachse ermittelt werden. So können beispielsweise die Auswirkungen von veränderten Produktionsparametern für ein Fadenprüfgut auf die Haarigkeitskennwerte beobachtet werden und anhand der Messwertergebnisse der Messvorrichtung die Produktionsparameter optimal eingestellt werden

Die Bildwiederholrate der Bildsensoreinrichtung kann verändert werden, bevorzugt wird die Bildwiederholrate an die vorliegende Geschwindigkeit des Fadenprüfguts angepasst, derart, dass die generierten Bilder nahtlos aneinander anschließen bzw. sich eine Überlappung zwischen den generierten Bildern ergibt.

Es kann jedoch auch vorgesehen sein, dass die Bildwiederholrate kleiner gewählt wird, so dass lediglich Bilder von Abschnitten des Fadenprüfguts generiert werden. Dieses weist den Vorteil auf, dass lediglich Teilbereiche der gesamten Fadenlänge des Fadenprüfgutes zur Ermittlung von statistisch verlässlichen Messwerten genutzt werden, was zu einer Reduktion der Datenmengen und des Auswertungsaufwands bei gleichbleibender Qualität der Messwerte führt.

Die Bildwiederholraten der Bildsensoreinrichtung können dabei im Bereich von 10 Hz bis 1000 Hz, bevorzugt im Bereich von 50 bis 200 Hz, liegen.

Gemäß einer besonders bevorzugten Ausführungsform ist es vorgesehen, dass die Bildsensoreinrichtung einen zweidimensionalen Bildsensor mit einer Vielzahl von matrixförmig angeordneten Bildelementen umfasst, wobei die Ebene des zweidimensionalen Bildsensors bevorzugt im Wesentlichen parallel zu der Fadenlängsachse sowie im Wesentlichen senkrecht zu der Einfallsrichtung der Lichtstrahlen der Beleuchtungseinrichtung angeordnet ist. Durch die im Wesentlichen parallele Ausrichtung des Bildsensors zu der Fadenlängsachse wird das Schattenbild des Fadenprüfgutes ohne Verschiebungen oder Verzerrungen auf der Bildebene der Sensoreinrichtung abgebildet, wodurch die Messgenauigkeit weiter erhöht und der Rechenaufwand der Auswertungseinheit reduziert werden kann.

Insbesondere kann es sich bei der Bildsensoreinrichtung um einen CMOS- oder einen CCD-Sensor handeln.

Die Bildelementgröße/Pixelgröße kann im Bereich von 1 µm bis 40 µm liegen. Die vorgenannten Bildelementgrößen ermöglichen eine gute bis sehr gute Abbildung der zu ermittelnden Fasern/Haare des Fadenprüfgutes und erhöhen die Genauigkeit der zu ermittelnden Kennwerte.

Im Rahmen der Datenreduktion und zur Erhöhung der Geschwindigkeit der Datenübertragung und Bildverarbeitung kann es vorgesehen werden, dass die Pixelgröße bzw. Größe der Einzelbildelemente durch die Kombination bzw. das gemittelte Auslesen mehrerer benachbarter Bildsegmente vergrößert und gleichzeitig die Pixelanzahl bzw. die Anzahl der Einzelbildelemente der Bildsensoreinrichtung virtuell verkleinert wird.

Für den Bildsensor kann insbesondere eine Anzahl von Bildelementen/Pixeln im Bereich von 0,1 Megapixel bis 40 Megapixel, bevorzugt im Bereich von 0,2 Megapixel bis 25 Megapixel, vorgesehen werden.

Erfindungsgemäß kann es vorgesehen sein, dass die Auswerteeinrichtung in mindestens einen von den mittels der Bildsensoreinrichtung ermittelten Bildern die Kanten des Kerns des Fadenprüfguts und/oder die Kanten der von dem Fadenkern des Fadenprüfguts abstehenden Einzelfasern detektiert.

In einer bevorzugten Ausführungsform der Erfindung kann es vorgesehen sein, dass der erste Abstand zwischen den Seitenwänden im Messkanalabschnitt im Bereich von 1-5 mm, bevorzugt im Bereich von 2-4 mm, liegt.

In einer weiterhin bevorzugten Ausführungsform umfasst die Beleuchtungseinrichtung eine Lichtquelle mit einer in Richtung des Messkanals im Strahlengang der Lichtquelle befindliche Blende.

Die Blende weist bevorzugt einen Durchmesser von weniger als 1 mm, besonders bevorzugt von weniger als 0,7 mm, auf, wobei die Wahl des Blendendurchmessers auf die bevorzugten Bereiche den Vorteil aufweist, dass dadurch die Lichtquelle unter einem kleinen Raumwinkel erscheint und damit das in der Bildsensoreinrichtung generierte Schattenbild des Fadenprüfguts schärfer dargestellt wird.

In einer weiterhin bevorzugten Ausführungsform ist es vorgesehen, dass sich in Bewegungsrichtung des Fadenprüfguts hinter dem Messkanalabschnitt zusätzlich ein hinterer Kanalabschnitt anschließt, wobei sich der Abstand zwischen den gegenüberliegenden Seitenwänden in dem hinteren Kanalabschnitt von einem ersten Abstand im Bereich der an den Messkanalabschnitt angrenzenden Eintrittsseite hin zu einem finalen Abstand im Bereich einer Austrittsseite des hinteren Kanalabschnitts erweitert, wobei der erste Abstand kleiner ist als der finale Abstand. Die Anordnung des hinteren Kanalabschnittes mit einem sich vergrößernden Abstand zwischen den Seitenwänden in Bewegungsrichtung des Fadens weist den Vorteil auf, dass sich der Druck im Bereich des hinteren Kanalabschnittes reduziert, wodurch ein Abfluss der Strömung aus dem Messkanal sichergestellt und ein Strömungsstau im Messkanal sowie die Bildung von Turbulenzen sicher verhindert werden.

Gemäß einem weiteren Aspekt der Erfindung kann diese alternativ zu der zuvor beschriebenen Ausführungsform dadurch gekennzeichnet sein, dass in Bewegungsrichtung des Fadenprüfgutes vor dem Messkanalabschnitt mit einer gleichbleibenden ersten Kanalbreite zusätzlich ein vorderer Kanalabschnitt ausgestaltet ist, wobei sich der Abstand zwischen den gegenüberliegenden Seitenwänden in dem vorderen Kanalabschnitt von einem initialen Abstand an einer Eintrittsseite hin zu dem ersten Abstand im Bereich der an den Messkanalabschnitt angrenzenden Austrittsseite des vorderen Kanalabschnitts verjüngt. Der initiale Abstand ist dabei größer als der erste Abstand. Die Eintrittsseite des vorderen Kanalabschnittes ist betrachtet in Fadenlängsrichtung dem Messkanalabschnitt abgewandt. Die gegenüberliegende Ausgestaltung der Bildsensoreinrichtung und der Beleuchtungseinrichtung ist bei diesem weiteren Aspekt der Erfindung optional, die weiteren bevorzugten Ausführungsformen entsprechen dabei den bevorzugten Ausführungsformen der Erfindung.

Die Erfindung betrifft weiterhin ein System umfassend eine erfindungsgemäße Vorrichtung zur Messung von Kennwerten eines Fadenprüfgeräts wie zuvor beschrieben in Kombination mit einem Fadenprüfgut, wobei die Vorrichtung an der Struktur des Fadenprüfgerätes gegenüber einem Fadenprüfgut schwenkbar und/oder verschiebbar angeordnet ist, wobei die Vorrichtung zur Messung von Haarigkeitskennwerten von einer Ruheposition, in welcher das Fadenprüfgut im Abstand zu der Messvorrichtung verläuft, in eine Messposition verlagerbar ist, in welcher das Fadenprüfgerät durch den Messkanal verläuft.

In einer alternativen Ausführungsform kann es ebenfalls vorgesehen sein, dass die erfindungsgemäße Vorrichtung ortsfest und damit in seiner Position unveränderlich gegenüber der Struktur des Fadenprüfgeräts angeordnet ist und das Fadenprüfgerät über mindestens eine relativ zu der erfindungsgemäßen Vorrichtung verlagerbare Fadenleiteinrichtung von einer Ruheposition in eine Messposition und umgekehrt überführbar ist.

Bevorzugte Ausführungsformen der Erfindung werden in den schematischen Figuren dargestellt.

Es zeigen:
- Fig. 1: einen Teilausschnitt des Messkanals der erfindungsgemäßen Vorrichtung zur Messung von Kennwerten eines Fadenprüfgutes in Schnittansicht,
- Fig. 2: eine Schnittansicht eines beispielhaften Fadenprüfgutes, sowie
- Fig. 3: eine perspektivische Ansicht der wesentlichen Merkmale eines erfindungsgemäßen Systems umfassend eine Vorrichtung zur Messung von Kennwerten des Fadenprüfgutes in Kombination weiterer Elemente eines Fadenprüfgeräts.

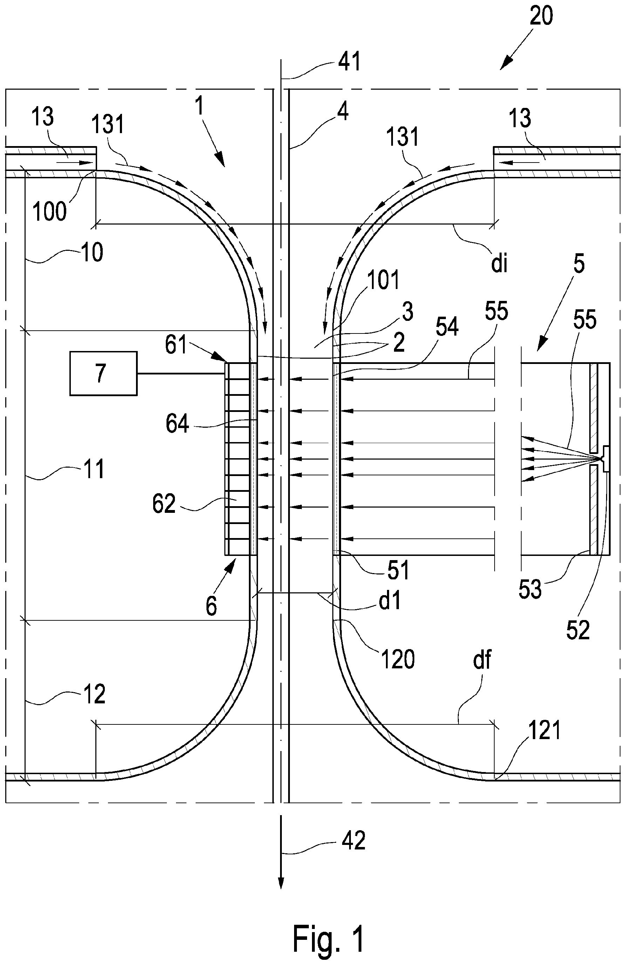

Die Fig. 1 zeigt eine beispielhafte Ausführungsform einer erfindungsgemäßen Vorrichtung 20 zur Messung von Kennwerten eines Fadenprüfgutes 4. Die erfindungsgemäße Vorrichtung 20 umfasst in der dargestellten Ausführungsform einen Messkanal 1, welcher in der dargestellten Bildebene seitlich begrenzt ist durch die zwei gegenüberliegenden Seitenwände 2 und in der Bildebene nach hinten durch eine Rückwand 3 begrenzt ist. In der Bildebene nach vorne ist der Messkanal 1 offen ausgestaltet. Wie dies in Fig. 1 gezeigt ist, ist der Messkanal 1 dabei derart ausgestaltet, dass das Fadenprüfgut 4 entlang dessen Fadenlängsachse 41 kontinuierlich durch den Messkanal durchführbar ist.

Die erfindungsgemäße Vorrichtung 20 weist weiterhin eine Beleuchtungseinrichtung 5 sowie eine elektronische Bildsensoreinrichtung 6 und eine Auswerteeinrichtung 7 für die Verarbeitung der Signale der Bildsensoreinrichtung 6 auf. Die Beleuchtungseinrichtung 5 und die Bildsensoreinrichtung 6 sind in einem ersten Abstand d1 relativ zueinander gegenüberliegend angeordnet, und bilden einen Abschnitt der gegenüberliegenden Seitenwände 2 eines ersten Abschnitts 11 des Messkanals 1. Die Beleuchtungseinrichtung 5 ist dabei derart angeordnet, dass diese unmittelbar ein Schattenbild des Fadenprüfgutes 4 auf die Bildsensoreinrichtung 6 wirft bzw. ein Schattenbild des Fadenprüfgutes 4 auf dieser unmittelbar abbildet. Das vorgenannte Schattenbild des Fadenprüfguts 4 wird dabei an die Auswerteeinrichtung 7 von der Bildsensoreinrichtung 6 übertragen zur Bestimmung mindestens eines Haarigkeitskennwertes und/oder Durchmesserwertes des Fadenprüfgutes 4. Wie dies ebenfalls der Fig. 1 entnehmbar ist, weisen sowohl die Beleuchtungseinrichtung 5 als auch die gegenüberliegende elektronische Bildsensoreinrichtung 6 mindestens eine dem Messkanal 1 zugewandte plane Oberfläche 54, 64 auf. Die vorgenannten gegenüberliegenden Oberflächen 54, 64 sind dabei im Wesentlichen parallel zu der Fadenlängsachse 41 des Fadenprüfgutes 4 angeordnet, derart, dass zumindest zwischen den Oberflächen 54, 64 der Bildsensoreinrichtung 6 und der Beleuchtungseinrichtung 5 ein Messkanalabschnitt mit einer gleichbleibenden ersten Messkanalbreite d1 ausgebildet ist, der mindestens einen Abschnitt des Messkanals 11 bildet.

In der dargestellten Ausführungsform der erfindungsgemäßen Vorrichtung 20 gemäß Fig. 1 ist in Bewegungsrichtung 42 des Fadenprüfguts 4 vor dem Messkanalabschnitt 11 zusätzlich ein weiterer vorderer Kanalabschnitt 10 ausgebildet. Der vordere Kanalabschnitt 10 ist dabei derart ausgestaltet, dass sich der Abstand zwischen den gegenüberliegenden Seitenwänden 2 in dem Bereich des vorderen Kanalabschnitts 10 von einem initialen Abstand di an der Eintrittsseite 100 hin zu dem ersten Abstand d1 im Bereich des an den Messkanalabschnitt 11 angrenzenden Austrittsseite 101 des vorderen Kanalabschnitts 10 verjüngt. Der vordere Kanalabschnitt 10 ist dabei zumindest durch die seitliche Begrenzung über die beiden gegenüberliegenden Seitenwände 2, deren Abstand sich in Bewegungsrichtung 42 des Fadenprüfguts 4 verjüngt, als Art einer konvergenten Düse in Bewegungsrichtung 42 ausgebildet. Die Eintrittsseite 100 des vorderen Kanalabschnitts 10 ist dabei dem Messkanalabschnitt 11 abgewandt. Der initiale Abstand di ist größer als der erste Abstand d1.

Im Bereich der Eintrittsseite 100 des Kanalabschnitts 10 sind in der dargestellten Ausführungsform an beiden gegenüberliegenden Seitenwänden 2 jeweils eine Ausströmöffnung 13 ausgebildet, über welche jeweils ein Gasvolumenstrom 131 parallel zu der Oberfläche der jeweiligen Seitenwand 2 im Bereich der Eintrittsseite 100 ausstoßbar ist. In der bevorzugten Ausführungsform wie in Fig. 1 dargestellt, sind dabei die Ausströmöffnungen 13 im Wesentlichen orthogonal zu der Längsachse 41 des Fadenprüfguts 4 angeordnet. Dabei ist der lokale Oberflächenlauf der Seitenwände 2 entsprechend der orthogonal ausgerichteten Ausströmöffnung 13 ausgerichtet, so dass in der Folge die Seitenwand 2 im Bereich der Eintrittsseite 100 ebenfalls im Wesentlichen orthogonal zu der Fadenlängsachse 41 des Fadenprüfguts 4 verläuft. Die beiden gegenüberliegenden Seitenwände 2 sind dabei im vorderen Kanalabschnitt 10 derart ausgebildet, dass diese im Bereich der Eintrittsseite 100 des vorderen Kanalabschnitts 10 im Wesentlichen orthogonal zu der Fadenlängsachse 41 und im Bereich der Austrittsseite 101 des vorderen Kanalabschnitts 10 im Wesentlichen parallel zu der Fadenlängsachse 41 verlaufen. Die beiden gegenüberliegenden Seitenwände 2 sind im Bereich des vorderen Kanalabschnitts 10 gekrümmt ausgestaltet, derart, dass diese einen konvexen Oberflächenverlauf gegenüber dem Messkanal 1 aufweisen. Aufgrund der Ausgestaltung der beiden Seitenwände 2 im vorderen Kanalabschnitt 20 wird der Gasvolumenstrom 131 aufgrund einer Wechselwirkung mit den Wänden 2,3, welche durch den sogenannten Coanda-Effekt zustande kommt, in den Messkanal 1 umgelenkt.

Die Beleuchtungseinrichtung 5 ist durch eine transparente Scheibe 51, welche eine im Wesentlichen plane Oberfläche 54 im Bereich des Messkanalabschnitts 11 ausbildet gegenüber dem Messkanal 1 abgegrenzt. Die Lichtquelle 52 der Beleuchtungseinrichtung 5 weist ferner einen definierten Abstand zu der transparenten Scheibe 51 auf, wobei der Abstand zwischen der Lichtquelle 52 zu dem Fadenprüfgut 4 groß in Relation zu dem Abstand zwischen Fadenprüfgut 4 und der elektronischen Bildsensoreinrichtung 6 gewählt ist. Die Bildsensoreinrichtung 6 ist durch einen zweidimensionalen Bildsensor 61 mit einer Vielzahl von matrixförmig angeordneten Bildelementen 62 gebildet, wobei aufgrund der Schnittansicht lediglich der Schnitt durch eine Reihe von Bildelementen 62 dargestellt ist. Die Ebene des zweidimensionalen Bildsensors 61 verläuft dabei im Wesentlichen parallel zu der Fadenlängsachse 41 sowie im Wesentlichen senkrecht zu der Einfallsrichtung der Lichtstrahlen 55 der Beleuchtungseinrichtung 5. Der Verlauf der Lichtstrahlen 55, welche von der Lichtquelle 52 der Beleuchtungseinrichtung 5 abgegeben bzw. ausgestrahlt werden, sind in der Fig. 1 schematisch als Pfeile 55 dargestellt.

Die Beleuchtungseinrichtung 5 mit der Lichtquelle 52 kann dabei eine in Richtung des Messkanals 1 im Strahlengang 55 der Lichtquelle 52 befindliche Blende 53 aufweisen. Zur besseren Darstellbarkeit ist der Abstand zwischen der Lichtquelle 52 und der transparenten Scheibe 51 verkürzt dargestellt.

In der Fig. 1 wurde der verbesserten Übersichtlichkeit halber auf die Darstellung der von dem Fadenkern 43 des Fadenprüfguts 4 abstehenden Einzelfasern bzw. Haare 44 verzichtet.

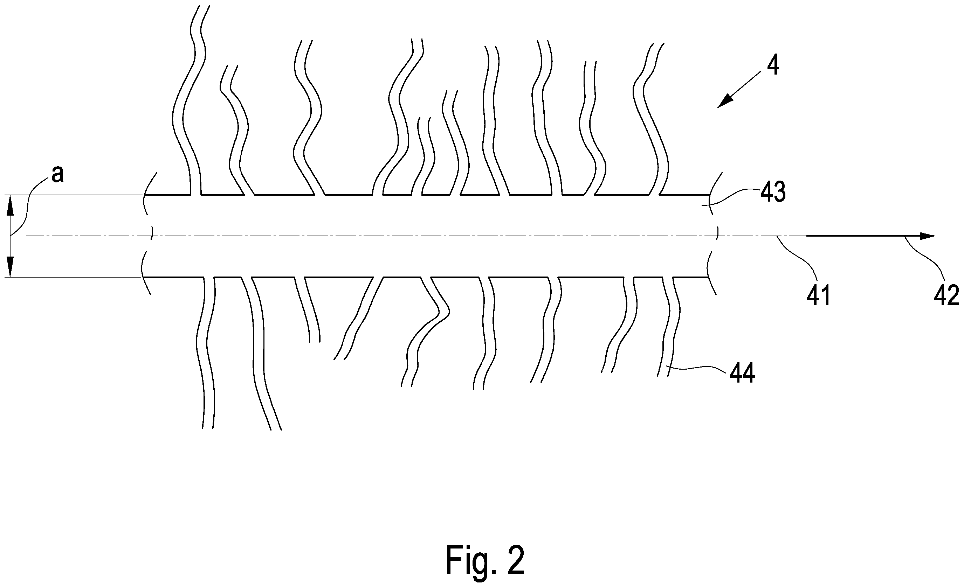

Fig. 2 zeigt eine detaillierte schematische Darstellung eines Schnitts eines Fadenprüfguts 4, von welchem Haarigkeitskennwerte oder Durchmesserwerte mit der erfindungsgemäßen Vorrichtung 20 gemessen werden. In der Fig. 2 sind lediglich die Umrisse und damit Kanten des Fadenprüfguts 4 wiedergegeben, wobei das Fadenprüfgut 4 eine Fadenlängsachse 41 aufweist. Das Fadenprüfgut 4 ist gebildet durch einen Fadenkern 43 mit einem Fadendurchmesser bzw. in der zweidimensionalen Darstellung einer Fadendicke a. Von dem vorgenannten Fadenkern 43 stehen eine Vielzahl von Einzelfasern bzw. Haaren 44 ab, deren Kennwerte mit der erfindungsgemäßen Vorrichtung 20 bestimmt werden.

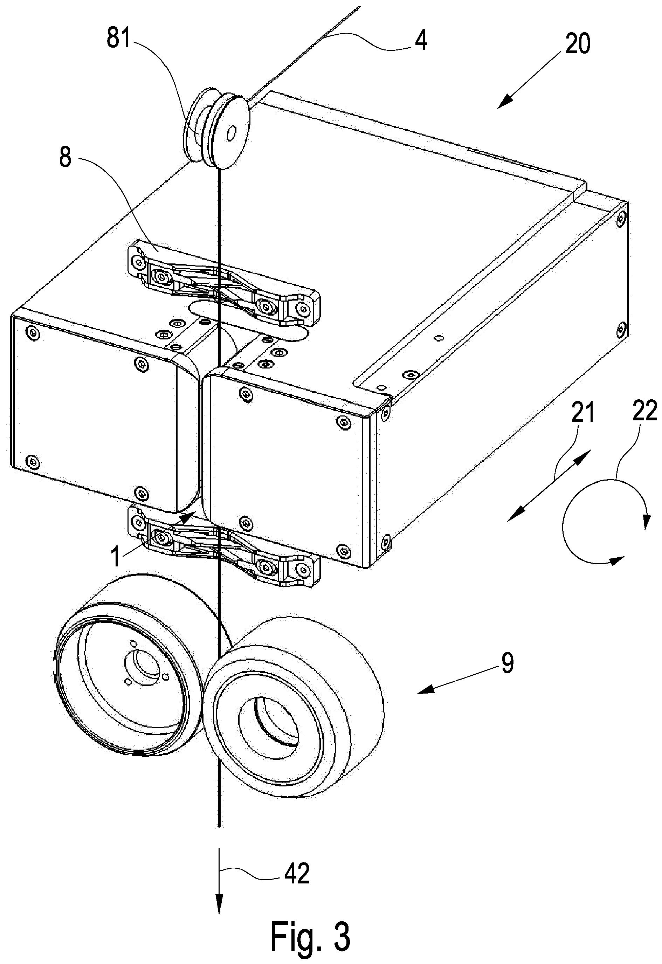

Die Fig. 3 zeigt eine perspektivische Ansicht eines erfindungsgemäßen Systems umfassend zumindest eine erfindungsgemäße Vorrichtung 20 zur Messung von Kennwerten eines Fadenprüfguts sowie ein Fadenprüfgerät, wobei von dem Fadenprüfgerät in der Fig. 3 lediglich die das Fadenprüfgut 4 leitenden Elemente im Bereich der erfindungsgemäßen Vorrichtung 20 dargestellt sind. Die erfindungsgemäße Vorrichtung 20 ist dabei an der nicht dargestellten Struktur des Fadenprüfguts derart angeordnet, dass diese relativ zu dem Fadenprüfgut 4 schwenkbar und/oder verschiebbar ausgestaltet ist. In der Fig. 3 ist eine Verschiebung schematisch als Doppelpfeil 21 sowie eine Verschwenkung mit Doppelpfeil 22 der Vorrichtung 20 relativ zu dem Fadenprüfgut 4 schematisch dargestellt. Alternativ oder zusätzlich zu der Verlagerung der Vorrichtung 20 relativ zu dem Fadenprüfgut 4 kann es ebenfalls vorgesehen sein, dass das Fadenprüfgut 4 in einer Ebene im Wesentlichen orthogonal zu der Fadenlängsachse 41 relativ zu dem Messkanal 1 bzw. der Vorrichtung 20 verlagert wird. In der Fig. 3 ist ebenfalls ein beispielhafter Verlauf des kontinuierlichen Fadenprüfguts 4 durch den Messkanal 1 der erfindungsgemäßen Vorrichtung 20 gezeigt, wobei sich die erfindungsgemäße Vorrichtung 20 gemäß Fig. 3 in der Messposition befindet. In Bewegungsrichtung 42 des Fadenprüfguts 4 vorgelagert zu der Messvorrichtung 20 befindet sich eine Fadenbremseinrichtung 81 sowie ein Fadenleitelement 8. Hinter dem Messkanal 1 der erfindungsgemäßen Vorrichtung 20 ist weiterhin eine Fadenfördereinrichtung 9 dargestellt.

## Patentansprüche

1. Vorrichtung zur Messung von Kennwerten eines Fadenprüfgutes (4) mit:
- einem Messkanal (1) mit zwei gegenüberliegenden Seitenwänden (2) und einer Rückwand (3) zur kontinuierlichen Durchführung des Fadenprüfgutes (4) entlang dessen Fadenlängsachse (41);
- einer Beleuchtungseinrichtung (5);
- einer elektronischen Bildsensoreinrichtung (6); sowie
- einer Auswerteeinrichtung (7) für die Signale der Bildsensoreinrichtung (6),
**dadurch gekennzeichnet, dass**
die Beleuchtungseinrichtung (5) und die Bildsensoreinrichtung (6) in einem ersten Abstand (d1) einander gegenüberliegend angeordnet sind, wobei die Beleuchtungseinrichtung (5) und die Bildsensoreinrichtung (6) zumindest abschnittsweise die gegenüberliegenden Seitenwände (2) eines Messkanalabschnittes (11) bilden, und dass die Beleuchtungseinrichtung (5) unmittelbar ein Schattenbild auf die Bildsensoreinrichtung (6) wirft, wobei mindestens ein Schattenbild des Fadenprüfgutes (4) an die Auswerteeinrichtung (7) zum Bestimmen mindestens eines Haarigkeitskennwertes und/oder Durchmesserwertes des Fadenprüfgutes (4) übertragen wird.

2. Vorrichtung nach Anspruch 1, **dadurch gekennzeichnet, dass** in Bewegungsrichtung (42) des Fadenprüfgutes (4) vor dem Messkanal (1) eine Bremseinrichtung (8) für das Fadenprüfgut (4) und/oder hinter dem Messkanal (1) eine Fördereinrichtung (9) für das Fadenprüfgut (4) angeordnet sind.

3. Vorrichtung nach Anspruch 1 oder 2, **dadurch gekennzeichnet, dass** die Beleuchtungseinrichtung (5) und die gegenüberliegende elektronische Bildsensoreinrichtung (6) jeweils eine dem Messkanal (1) zugewandte plane Oberfläche (54, 64) aufweisen, wobei die gegenüberliegenden Oberflächen (54, 64) im Wesentlichen parallel zu der Fadenlängsachse (41) angeordnet sind, derart, dass zumindest zwischen den Oberflächen (54, 64) der Bildsensoreinrichtung (6) und der Beleuchtungseinrichtung (5) ein Messkanalabschnitt mit einer gleichbleibenden ersten Kanalbreite (d1) gebildet ist.

4. Vorrichtung nach einem der Ansprüche 1 bis 3, **dadurch gekennzeichnet, dass** in Bewegungsrichtung (42) des Fadenprüfgutes (4) vor dem Messkanalabschnitt (11) zusätzlich ein vorderer Kanalabschnitt (10) vorgesehen ist, wobei sich der Abstand zwischen den gegenüberliegenden Seitenwänden (2) in dem vorderen Kanalabschnitt (10) von einem initialen Abstand (di) an einer Eintrittsseite (100) hin zu dem ersten Abstand (d1) im Bereich des an den Messkanalabschnitt (11) angrenzenden Austrittsseite (101) des vorderen Kanalabschnittes (10) verjüngt.

5. Vorrichtung nach Anspruch 4, **dadurch gekennzeichnet, dass** im Bereich der Eintrittsseite (100) des vorderen Kanalabschnittes (10) an mindestens einer der gegenüberliegenden Seitenwände (2) eine Ausströmöffnung (13) ausgebildet ist, über welche ein Gasvolumenstrom (131) parallel zu der Oberfläche der jeweiligen Seitenwand (2) im Bereich der Eintrittsseite (100), bevorzugt orthogonal zu der Längsachse (41) des Fadenprüfgutes (4), ausstoßbar ist.

6. Vorrichtung nach Anspruch 5, **dadurch gekennzeichnet, dass** der Verlauf von mindestens einer der Wände (2, 3) im Bereich des vorderen Kanalabschnittes (10) derart ausgestaltet ist, dass diese eine Wechselwirkung mit dem über die Ausströmöffnung (13) ausgestoßenen Gasvolumenstrom (131) ausübt, derart, dass der Gasvolumenstrom (131) entlang der mindestens einen entsprechend ausgestalteten Wand (2, 3) des vorderen Kanalabschnittes (10) von einer zunächst schräg, bevorzugt orthogonal, zu der Fadenlängsachse (41) verlaufenden Bewegungsrichtung im Bereich der Ausströmöffnung (13) in eine im Wesentlichen parallel zu der Fadenlängsachse (41) verlaufende Bewegungsrichtung im Bereich des Austrittsendes (101) des vorderen Kanalabschnittes (10) umgelenkt wird.

7. Vorrichtung nach einem der Ansprüche 4 bis 6, **dadurch gekennzeichnet, dass** mindestens eine der Wände (2, 3) im Bereich der Eintrittsseite (100) des vorderen Kanalabschnittes (10) schräg, bevorzugt orthogonal, zu der Fadenlängsachse (41) und im Bereich der Austrittsseite (101) des vorderen Kanalabschnittes (10) im Wesentlichen parallel zu der Fadenlängsachse (41) verläuft.

8. Vorrichtung nach einem der Ansprüche 4 bis 7, **dadurch gekennzeichnet, dass** mindestens eine der Wände (2, 3) im Bereich des vorderen Kanalabschnittes (10) gekrümmt ausgestaltet ist, wobei die mindestens eine gekrümmte Wand (2, 3) einen konvexen Oberflächenverlauf aufweist.

9. Vorrichtung nach einem der Ansprüche 4 bis 8, **dadurch gekennzeichnet, dass** das pro definierter Zeitdauer ausgestoßene Volumen des über die mindestens eine Ausströmöffnung (13) ausgestoßenen Gases (131) veränderbar ist.

10. Vorrichtung nach einem der vorhergehenden Ansprüche, **dadurch gekennzeichnet, dass** die Beleuchtungseinrichtung (5) durch eine transparente Scheibe (51) und eine in einem definierten Abstand zu der transparenten Scheibe (51) angeordneten Lichtquelle (52) ausgebildet ist.

11. Vorrichtung nach einem der vorhergehenden Ansprüche, **dadurch gekennzeichnet, dass** die Bildsensoreinrichtung (6) eine Mehrzahl an Bildern von dem Fadenprüfgut (4) mit einer definierten Bildwiederholrate aufnimmt und zur Bestimmung von Haarigkeitskennwerten an die Auswerteeinrichtung (7) überträgt.

12. Vorrichtung nach einem der vorhergehenden Ansprüche, **dadurch gekennzeichnet, dass** die Bildsensoreinrichtung (6) einen zweidimensionalen Bildsensor (61) mit einer Vielzahl von matrixförmig angeordneten Bildelementen (62) umfasst, wobei die Ebene des zweidimensionalen Bildsensors (61) bevorzugt im Wesentlichen parallel zu der Fadenlängsachse (41) sowie im Wesentlichen senkrecht zu der Einfallsrichtung der Lichtstrahlen der Beleuchtungseinrichtung (5) angeordnet ist.

13. Vorrichtung nach einem der vorhergehenden Ansprüche, **dadurch gekennzeichnet, dass** die Auswerteeinrichtung (7) in mindestens einem von den mittels der Bildsensoreinrichtung (6) ermittelten Bildern die Kanten des Kerns des Fadenprüfgutes (4) und/oder die Kanten der von dem Fadenkern (43) des Fadenprüfgutes (4) abstehenden Einzelfasern (44) detektiert.

14. Vorrichtung nach einem der vorhergehenden Ansprüche, **dadurch gekennzeichnet, dass** die Beleuchtungseinrichtung (5) eine Lichtquelle (52) mit einer in Richtung des Messkanals (1) im Strahlengang (55) der Lichtquelle (52) befindliche Blende (53) umfasst.

15. System umfassend eine Vorrichtung gemäß einem der Ansprüche 1 bis 14 und ein Fadenprüfgerät, wobei die Vorrichtung (20) an der Struktur des Fadenprüfgeräts gegenüber einem Fadenprüfgut (4) schwenkbar und/oder verschiebbar angeordnet ist, wobei die Vorrichtung (20) zur Messung von Haarigkeitskennwerten von einer Ruheposition, in welcher das Fadenprüfgut (4) im Abstand zu der Messvorrichtung (20) verläuft, in eine Messposition verlagerbar ist, in welcher das Fadenprüfgut durch den Messkanal (1) verläuft.
